# FASCICULE DE BREVET EUROPEEN

(11) **EP 0 428 460 B1**
(45) Date de publication et mention de la délivrance du brevet: **29.03.1995**
(21) Numéro de dépôt: 90440094.2
(22) Date de dépôt: 06.11.1990
(51) Int. Cl.: C07F 7/18, C07C 45/51, C07C 47/273, C07C 47/277

(54) **Nouveaux diénoxysilanes, leur procédé d'obtention, et nouveaux aldéhydes alpha-éthyléniques alpha-halogénés auxquels ils donnent accès**
Dienoxysilane, Verfahren zu ihrer Herstellung und alpha-ethylenische alpha-halogenierte Aldehyde davon erreichbar
Dienoxysilanes, methods for their preparation and alpha-ethylenic alpha-halo-aldehydes accessible therefrom

(30) Priorité: 09.11.1989 FR 8914882
(43) Date de publication de la demande: 22.05.1991
(73) Titulaire: UNIVERSITE DE ROUEN, F-76134 Mont-Saint-Aignan Cédex (FR)
(72) Inventeur: Combret, Jean-Claude, F-76000 Rouen (FR); Klein, Jean-Louis, F-76130 Mont Saint-Aignan (FR); Le Gailliard, Joel, F-76000 Rouen (FR)
(74) Mandataire: Dénoyez, Hubert

(56) Documents cités:
- EP-A- 0 353 732
- GB-A- 1 541 972
- GB-A- 1 591 968
- SYNTHESIS, no. 1, janvier 1983, pages 1-28, Georg Thieme Verlag, Stuttgart, DE;P. BROWNRIDGE: "Sylil enol ethers in synthesis - part I"
- SYNTHESIS, février 1983, pages 85-105, Georg Thieme Verlag, Stuttgart, DE; P.BROWNRIDGE: "Silyl enol ethers in synthesis - Part II"

## Description

La présente invention a pour objet de nouveaux diénoxy-silanes, leur procédé d'obtention, les nouveaux aldéhydes α-éthyléniques α-halogénés auxquels ils donnent accès et le procédé d'obtention de ces nouveaux aldéhydes α-éthyléniques α-halogénés.

Les diénoxysilanes fonctionnels qui constituent le premier objet de la présente invention sont des intermédiaires de synthèse d'un grand intérêt, car ouvrant un nouvel accès à des macrolides tels que la pyrénophorine, la norpyrénophorine ou la vermiculine, qui présentent des propriétés fongicides.

Les diénoxysilanes qui constituent le premier objet de la présente invention peuvent être représentés par la formule générale : 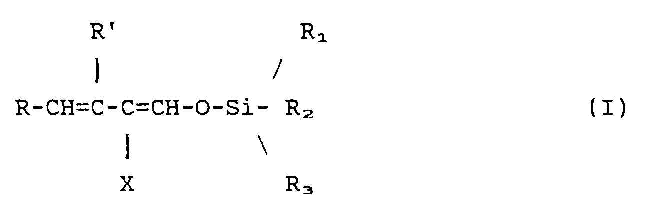
dans laquelle R désigne un atome d'hydrogène ou un radical alkyle linéaire renfermant de 1 à 4 atomes de carbone, R′ désigne un atome d'hydrogène ou un radical méthyle, R₁, R₂ et R₃ désignent chacun un radical alkyle renfermant de 1 à 4 atomes de carbone ou un radical aryle et X désigne un atome d'halogène, un radical dialkylamino dont les groupes alkyle renferment chacun de 1 à 4 atomes de carbone ou un radical pyrrolidino , pipéridino ou morpholino, avec la condition que R′ ne peut être un radical méthyle que lorsque R est un atome d'hydrogène.

Les diénoxysilanes de l'invention peuvent être obtenus à partir d'un trialkylchlorosilane et d'un aldéhyde α-éthylénique α-substitué, selon la réaction générale suivante : 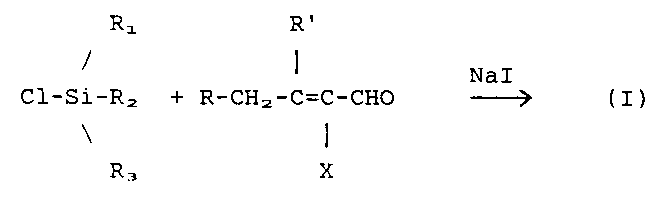
dans laquelle les différents substituants répondent à la même définition que précédemment.

Cette réaction, connue pour l'obtention de diénoxysilanes non fonctionnels, doit toutefois être conduite en milieu anhydre maintenu jusqu'à l'isolement des diénoxysilanes, en raison de la sensibilité à l'hydrolyse de ces composés.

Le procédé d'obtention des diénoxysilanes de l'invention comprend ainsi, dans une première étape, la mise en oeuvre de cette réaction, qui s'effectue à température ambiante, sous atmosphère inerte et sous agitation, dans un solvant anhydre tel que l'acétonitrile et en présence d'une base faible qui peut être un amine tertiaire comme la triéthylamine, la pyridine, la N-éthylmorpholine ou la 4-diméthylaminopyridine.

Dans cette première étape du procédé selon l'invention, le trialkylchlorosilane et l'aldéhyde α-éthylénique α-substitué sont mis en oeuvre en quantités sensiblement équimoléculaires, de même que la triéthylamine et l'iodure de sodium, qui sont toutefois mis en oeuvre en quantités molaires sensiblement supérieures.

La deuxième étape du procédé selon l'invention consiste en l'extraction du diénoxysilane du mélange réactionnel, cette extraction pouvant être réalisée à l'aide d'un solvant apolaire tel que l'éther de pétrole, le pentane, l'hexane ou le cyclohexane.

Le rendement de la réaction est de l'ordre de 75 à 90%, et les produits obtenus sont suffisamment purs pour ne pas nécessiter de purification ultérieure.

Parmi les diénoxysilanes de l'invention, ceux qui sont α-halogénés présentent la propriété de réagir sélectivement en 4 avec des composés électrophiles, et cette propriété peut être avantageusement utilisée pour obtenir les aldéhydes α-éthyléniques α-halogénés qui constituent un autre objet de la présente invention.

Les aldéhydes α-éthyléniques α-halogénés selon l'invention répondent à la formule générale : 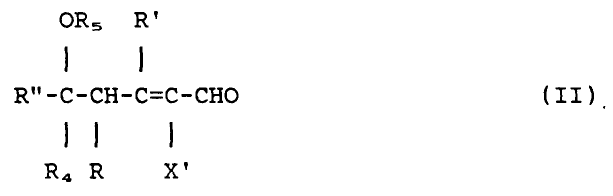
dans laquelle R et R′ répondent à la même définition que précédemment, X′ désigne un atome d'halogène, R˝ un radical alkyle, aryle ou hétéroaryle, un radical alkyléthylénique ou aryléthylénique ou un radical méthoxy ou éthoxy, R₄ un atome d'hydrogène ou un radical méthyle et R₅ un atome d'hydrogène ou un radical méthyle ou éthyle.

Les aldéhydes α-éthyléniques α-halogénés de formule (II) peuvent être obtenus selon un procédé qui constitue également un objet de la présente invention, par action d'un aldéhyde, d'un acétal ou d'un orthoformiate sur un diénoxysilane halogéné de formule générale : 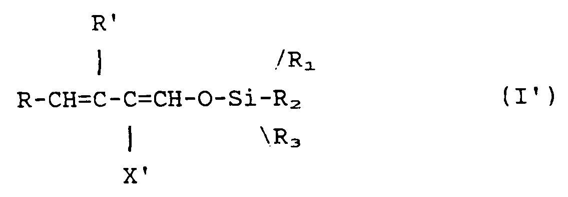
dans laquelle les différents substituants répondent aux mêmes définitions que précédemment.

La réaction est effectuée à une température de O° à 20°C dans un solvant anhydre comme le dichlorométhane, sous atmosphère inerte et en présence d'un catalyseur acide de LEWIS qui peut être le tétrachlorure de titane, le dichlorure de zinc ou le dibromure de zinc, les produits de départ étant mis en oeuvre en quantités sensiblement équimoléculaires.

Selon un premier mode de réalisation du procédé selon l'invention, on soumet un diénoxysilane de formule (I') à l'action d'un aldéhyde de formule générale R''CHO, la réaction conduisant à l'obtention d'un aldéhyde α-éthylénique α-halogéné de formule : 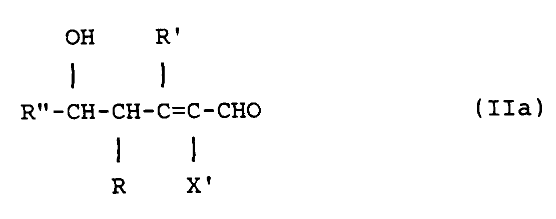
dans laquelle R, R', R'' et X' répondent aux mêmes définitions que précédemment.

Le temps de réaction varie de 2 à 3 heures à O°C ou à température ambiante, et le rendement en produit (IIa) est de 80 à 90 %.

Il convient de noter que selon la structure du diénoxysilane (I') et la nature de l'aldéhyde mis en oeuvre, le composé (IIa) peut être obtenu en mélange avec le siloxane correspondant, et dans certains cas la réaction conduit à l'obtention de ce seul siloxane, dont l'hydrolyse fournit le composé (IIa).

Selon un second mode de réalisation du procédé selon l'invention, on soumet un diénoxysilane de formule (I') à l'action d'un acétal d'aldéhyde ou de cétone, de formule générale: 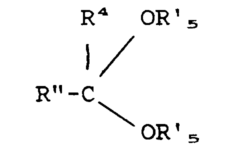
cette réaction conduisant à l'obtention d'un aldéhyde α-éthylénique α-halogéné de formule : 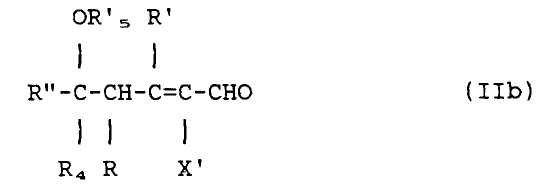
dans laquelle les substituants R, R', R'', R₄ et X' répondent à la même définition que précédemment, et R'₅ désigne un radical méthyle ou éthyle.

Le temps de réaction varie de 10 minutes à 4 heures à température ambiante, et le rendement en produit (IIb) est de l'ordre de 80 %.

Selon un troisième mode de réalisation du procédé selon l'invention, on soumet un diénoxysilane de formule (I') à l'action d'un orthoformiate de formule générale HC (OR'₅)₃, cette réaction conduisant à l'obtention d'un aldéhyde α-éthylénique α-halogéné de formule 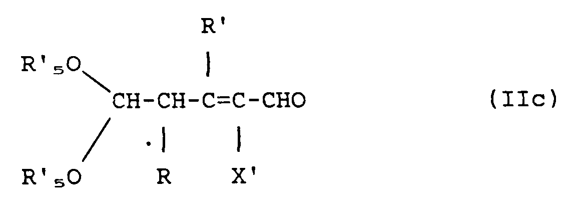
dans laquelle les substituants répondent aux mêmes définitions que précédemment.

Le temps de réaction est de l'ordre de 24 heures à température ambiante, et le rendement en produit (IIc) est de l'ordre de 70 %.

Dans tous les cas, l'aldéhyde α-éthylénique α-halogéné est isolé du milieu réactionnel après addition d'eau, la phase aqueuse étant extraite à l'aide de dichlorométhane et la phase organique étant séchée sur sulfate de magnésium anhydre avant évaporation du solvant conduisant à l'obtention de l'aldéhyde.α-éthylénique α-halogéné.

Les produits obtenus peuvent être ensuite distillés ou purifiés par chromatographie sur colonne.

Les aldéhydes α-éthyléniques α-halogénés de formule II sont également des intermédiaires de synthèse importants, constituant l'une des étapes de la nouvelle voie d'accès aux macrolides ouverte par les diénoxysilanes de formule I.

Les exemples qui suivent sont fournis à titre d'illustration de la présente invention, étant bien entendu qu'ils ne présentent aucun caractère limitatif vis-à-vis de l'invention.

### EXEMPLE 1

### Préparation du 2-bromo-1-triméthylsiloxybuta-1,3-diène.

On place 5,5 g d'iodure de sodium dans un creuset en acier inoxydable et l'on chauffe à l'aide d'un bec Bunsen à 200°C pendant 15 mn en agitant constamment le solide. On introduit 4,5 g (30 mmoles) de cet iodure de sodium chaud dans 40 cm³ d'acétonitrile séché par distillation sur hydrure de calcium.

D'autre part on introduit, dans un récipient de 250 ml, 2,98 g (20 mmoles) de 2-bromobut-2-ènal et 3 g (30 mmoles) de triéthylamine. A température ambiante, sous atmosphère d'azote et sous agitation, on ajoute 1,63 g (30 mmoles) de chlorotriméthylsilane puis goutte à goutte la solution d'iodure de sodium dans l'acétonitrile préalablement préparée. Après 90 mn de réaction, on ajoute 60 cm³ d'essence G anhydre (éther de pétrole 40-60) et on agite pendant 30 mn. On filtre le précipité formé sous atmosphère d'azote puis on le lave avec 2 fois 30 ml d'essence G anhydre. On recueille la phase supérieure du filtrat (essence G) et l'on extrait la phase inférieure (acétonitrile) avec 3 fois 30 ml d'essence G. On rassemble les fractions essence G, auxquelles on ajoute 5 g de sulfate de magnésium anhydre. On filtre sur verre fritté et élimine le solvant par distillation sous vide (évaporateur rotatif) sous atmosphère inerte. On dilue le résidu obtenu dans 80 cm³ d'éther éthylique anhydre, filtre le précipité et évapore le solvant sous atmosphère d'azote. On obtient 3,76 g de produit, ce qui représente un rendement de 85 %. Le taux de pureté (CPG) du produit obtenu est de 98 % et sa structure est confirmée par analyse RMN et spectrométrie de masse.

### EXEMPLE 2.

### Préparation du 2-morpholino-1-triméthylsiloxybut-1,3-diène.

En procédant de la même manière qu'à l'exemple 1, et avec les mêmes proportions des différents produits, on obtient, à partir de 3,1 g de 2-morpholinobut-2-ènal, 3,4 g de produit, ce qui représente un rendement de 75 %. Le taux de pureté du produit obtenu est de 98 % (CPG), et sa structure est confirmée par analyse RMN.

### EXEMPLE 3.

### Préparation du 2-bromo-5-éthoxyhex-2-ènal.

Dans un réacteur de 100 ml parcouru par un courant d'azote, on introduit une solution de 2,21 g (10 mmoles) de 2-bromo-1-triméthylsiloxybut-1,3-diène dans 30 ml de dichlorométhane anhydre. On ajoute lentement 1,18 g (environ 11 mmoles) de 1,1-diéthoxyéthane puis 100 mg de bromure de zinc anhydre. Après 10 mn de réaction, on ajoute 20 ml d'eau, on recueille la phase organique et extrait la phase aqueuse avec 20 ml de dichlorométhane. On rassemble les fractions dichlorométhane que l'on sèche avec 5 g de sulfate de magnésium anhydre. On élimine le solvant par évaporation sous vide et l'on distille le résidu, la température d'ébullition du produit étant de 66°C sous O,15 mm Hg. On obtient 1,72 g de produit, ce qui représente un rendement de 78%. La structure du produit obtenu est confirmée par analyse RMN et spectrométrie de masse.

### EXEMPLE 4.

### Préparation du 2-bromo-5-éthoxy-7-phénylhept-2,6-diènal.

On part de 2,21 g de 2-bromo-1-triméthylsiloxybut-1,3-diène et 2,06 g de 3,3-diéthoxyl-1-phénylprop-1-ène et on procède de la même manière qu'à l'exemple 3, à ceci près que la réaction est prolongée pendant 2 heures.

Le produit de la réaction est purifié par chromatographie en phase liquide sur colonne de silice, l'éluant étant un mélange essence G-acétate d'éthyle (90/10 en volume).

On obtient 2,63 g de produit, ce qui représente un rendement de 85 %. La structure du produit est confirmée par analyse RMN.

### EXEMPLE 5.

### Préparation du 2-bromo-5,5-diméthoxypent-2-ènal.

On part de 2,21 g de 2-bromo-1-triméthylsiloxybut-1,3-diène, de 1,06 g d'orthoformiate de méthyle (triméthoxyméthane) et 0,2 g de chlorure de zinc et on procède dans les mêmes conditions qu'à l'exemple 3, le temps de réaction étant de 24 heures.

On obtient 1,61 g de produit brut composé de 90 % de 2-bromo-5,5-diméthoxypent-2-ènal et de 10 % de 2-bromo-1,1,5,5-tétraméthoxypent-2-ène, ce qui correspond à un rendement de 72 %. La structure du produit obtenu est confirmée par analyse RMN et spectrométrie de masse.

### EXEMPLE 6.

### Préparation du 2-bromo-5-hydroxyhex-2-ènal.

On part de 2,21 g de 2-bromo-1-triméthylsiloxybut-1,3-diène, de 0,44 g d'éthanal et 0,1 g de bromure de zinc et on procède de la même manière qu'à l'exemple 3, le temps de réaction étant de 3 heures.

On obtient 1,74 g de produit brut (pureté 95 %), ce qui représente un rendement de 90.%. La structure du produit obtenu est confirmée par analyse RMN et IR.

## Revendications

1. Nouveaux diénoxysilanes répondant à la formule générale: 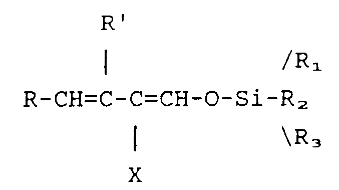 dans laquelle R désigne un atome d'hydrogène ou un radical alkyle linéaire renfermant de 1 à 4 atomes de carbone, R' désigne un atome d'hydrogène ou un radical méthyle, R₁, R₂, R₃ désignent chacun un radical alkyle renfermant de 1 à 4 atomes de carbone ou un radical aryle et X désigne un atome d'halogène, un radical dialkylamino dont les groupes alkyle renferment chacun de 1 à 4 atomes de carbone, ou un radical pyrrolidino, pipéridino ou morpholino, avec la condition que R' ne peut être un radical méthyle que lorsque R est un atome d'hydrogène.

2. Procédé d'obtention des diénoxysilanes qui font l'objet de la revendication 1, caractérisé en ce qu'il consiste à soumettre un aldéhyde α-éthylénique α-substitué de formule 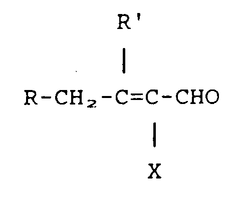 à l'action conjuguée d'iodure de sodium et d'un chlorosilane de formule 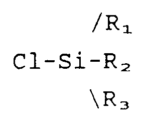 les différents substituants de ces deux formules répondant aux définitions indiquées dans la revendication 1, la réaction étant effectuée à température ambiante, en milieu anhydre , sous atmosphère inerte et sous agitation, en présence d'une base faible telle qu'une amine tertiaire, et le diénoxysilane étant isolé du milieu réactionnel par extraction à l'aide d'un solvant apolaire approprié.

3. Procédé selon la revendication 2, caractérisé en ce que la réaction est effectuée dans l'acétonitrile anhydre en présence de triéthylamine.

4. Procédé selon la revendication 2 ou la revendication 3, caractérisé en ce que le diénoxysilane est isolé du mélange réactionnel par extraction à l'aide d'un solvant choisi dans le groupe formé par l'éther de pétrole, le pentane, l'hexane et le cyclohexane.

5. Nouveaux aldéhydes α-éthyléniques α-halogénés obtenus à partir des diénoxysilanes qui font l'objet de la revendication 1 et répondant à la formule générale : 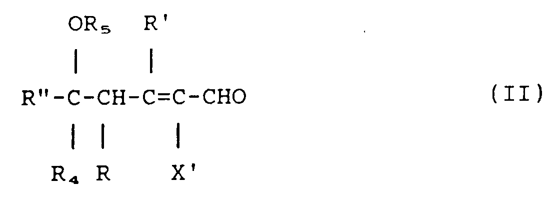 dans laquelle R représente un atome d'hydrogène ou un radical alkyle linéaire renfermant de 1 à 4 atomes de carbone, R' représente un atome d'hydrogène ou un radical méthyle, X' représente un atome d'halogène, R'' un radical alkyle, aryle ou hétéroaryle, un radical alkyléthylénique ou aryléthylénique ou un radical méthoxy ou éthoxy, R₄ un atome d'hydrogène ou un radical méthyle et R₅ un atome d'hydrogène ou un radical méthyle ou éthyle, avec la condition que R' ne peut être un radical méthyle que lorsque R est un atome d'hydrogène.

6. Procédé d'obtention des aldéhydes α-éthyléniques α-halogénés qui font l'objet de la revendication 5, caractérisé en ce que l'on soumet un aldéhyde, un acétal ou un orthoformiate à l'action d'un diénoxysilane halogéné de formule : 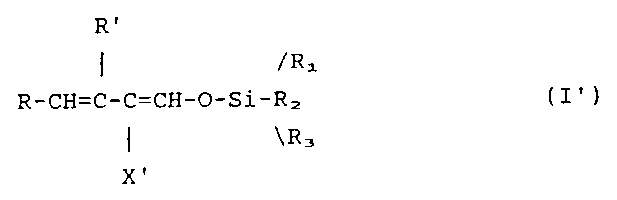 dans laquelle X' représente un atome d'halogène et les substituants R, R', R₁, R₂ et R₃ répondent aux définitions indiquées dans la revendication 1,
la réaction étant effectuée à une température de O°C à 20°C dans un solvant anhydre, sous atmosphère inerte et en présence d'un catalyseur acide de Lewis, et l'aldéhyde α-éthylénique α-halogéné étant isolé du milieu réactionnel par addition d'eau suivie de séparation de la phase aqueuse, la phase organique étant séchée sur sulfate de magnésium anhydre avant évaporation du solvant.

7. Procédé selon la revendication 6, caractérisé en ce que la réaction est effectuée dans du dichlorométhane anhydre en présence d'un catalyseur choisi dans le groupe formé par le tétrachlorure de titane, le dichlorure de zinc et le dibromure de zinc.

8. Procédé selon la revendication 6 ou la revendication 7 conduisant à l'obtention d'un aldéhyde α-éthylénique α-halogéné de formule : 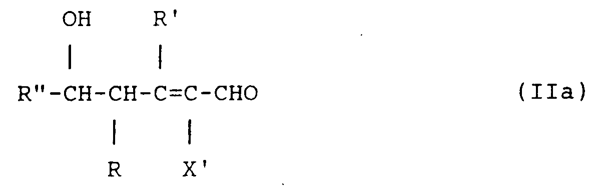 dans laquelle les substituants R, R', R'' et X' répondent aux définitions indiquées dans la revendication 5, caractérisé en ce que l'on soumet un diénoxysilane de formule (I') à l'action d'un aldéhyde de formule R''CHO, le produit obtenu étant si nécessaire soumis à une hydrolyse visant à transformer le siloxane correspondant en aldéhyde α-éthylénique α-halogéné.

9. Procédé selon la revendication 6 ou la revendication 7 conduisant à l'obtention d'un aldéhyde α-éthylénique α-halogéné de formule : 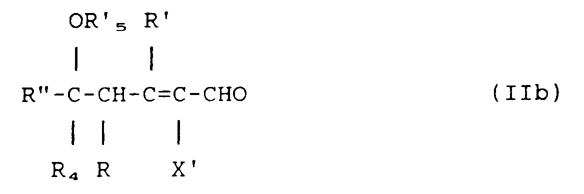 dans laquelle R'₅ désigne un radical méthyle ou éthyle et les substituants R, R', R'', R₄ et X' répondent aux définitions indiquées dans la revendication 5, caractérisé en ce que l'on soumet un diénoxysilane de formule (I') à l'action d'un acétal de formule : 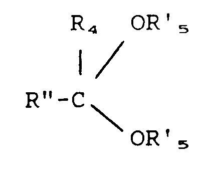 dans laquelle R'₅ désigne un radical méthyle ou éthyle et les substituants R'' et R₄ répondent aux définitions indiquées dans la revendication 5.

10. Procédé selon la revendication 6 ou la revendication 7, conduisant à l'obtention d'un aldéhyde α-éthylénique α-halogéné de formule : 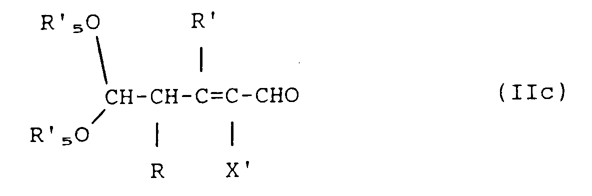 dans laquelle R'₅ désigne un radical méthyle ou éthyle et les substituants R, R' et X' répondent aux définitions indiquées dans la revendication 5, caractérisé en ce que l'on soumet un diénoxysilane de formule (I') à l'action d'un orthoformiate de formule HC(OR'₅)₃ où R'₅ désigne un radical méthyle ou éthyle.

## Claims

1. New dienoxysilanes having the general formula 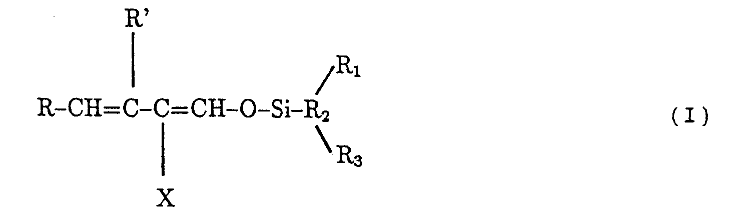 in which R is a hydrogen atom or a linear alkyl radical containing 1 to 4 carbon atoms, R' is a hydrogen atom or a methyl radical, each of R₁, R₂, R₃ designates an alkyl radical containing 1 to 4 carbon atoms or an aryl radical and X is a halogen atom, a dialkylamino radical each of the alkyl groups of which contains 1 to 4 carbon atoms, or a pyrrolidino, piperidino or morpholino radical, with the condition that R' can be a methyl radical only when R is a hydrogen atom.

2. A method of obtaining dienoxysilanes of claim 1, characterized by the fact that it consists in subjecting an α-substituted α-ethylene aldehyde of the formula 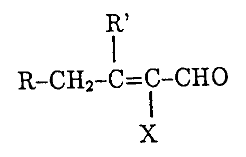 to the combined action of sodium iodide and a chlorosilane of the formula 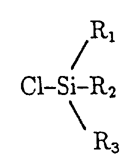 the different substituents of these two formulas satisfying the definitions indicated in claim 1, the reaction being carried out at room temperature, in anhydrous medium, in an inert atmosphere and with agitation, in the presence of a weak base such as a tertiary amine, and the dienoxysilane being isolated from the reaction medium by extraction with a suitable nonpolar solvent.

3. A method according to claim 2, characterized by the fact that the reaction is carried out in anhydrous acetonitrile in the presence of triethylamine.

4. A method according to claim 2 or claim 3, characterized by the fact that the dienoxysilane is isolated from the reaction mixture by extraction with a solvent selected from the group consisting of petroleum ether, pentane, hexane and cyclohexane.

5. New α-halo α-ethylene aldehydes obtained from dienoxysilanes of claim 1 and having the general formula : 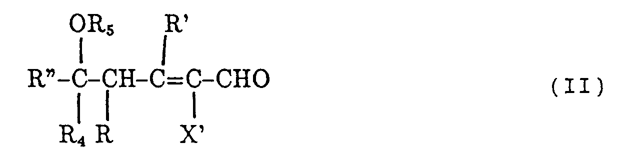 in which R is a hydrogen atom or a linear alkyl radical containing from 1 to 4 carbon atoms, R' is a hydrogen atom or a methyl radical, X' is a halogen atom, R'' is an alkyl, aryl or heteroaryl radical, an alkylethylene or arylethylene radical, or a methoxy or ethoxy radical, R₄ is a hydrogen atom or a methyl radical and R₅ is a hydrogen atom or a methyl or ethyl radical, with the condition that R' can be a methyl radical only when R is a hydrogen atom.

6. A method of obtaining α-halo α-ethylene aldehydes of claim 5, characterized by subjecting an aldehyde, an acetal or an orthoformate to the action of a halogenated dienoxysilane of the formula 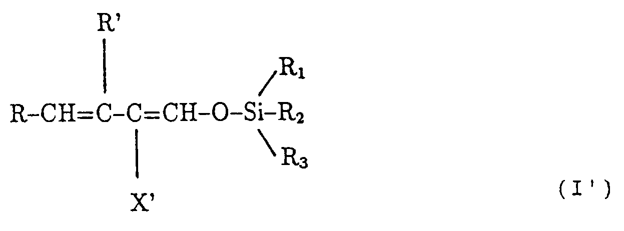 in which X' represents a halogen atom and the substituents R, R', R₁, R₂ and R₃ satisfy the definitions given in claim 1, the reaction being carried out at a temperature of 0°C to 20°C in an anhydrous solvent, in an inert atmosphere and in the presence of a Lewis acid catalyst, and the α-halo α-ethylene aldehyde being isolated from the reaction medium by addition of water, followed by separation of the aqueous phase, the organic phase being dried over anhydrous magnesium sulfate before evaporation of the solvent.

7. A method according to claim 6, characterized by the fact that the reaction is carried out in anhydrous dichloromethane in the presence of a catalyst selected from the group consisting of titanium tetrachloride, zinc dichloride and zinc dibromide.

8. A method according to claim 6 or claim 7 leading to the obtention of an α-halo α-ethylene aldehyde of the formula 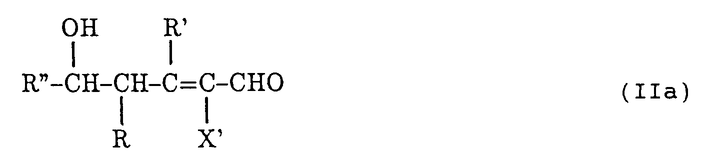 in which the substituents R, R', R'' and X' satisfy the definitions given in claim 5, characterized by subjecting a dienoxysilane of formula I' to the action of an aldehyde of the formula R''CHO, the product obtained being, if necessary, subjected to hydrolysis for transforming the corresponding siloxane into α-halo α-ethylene aldehyde.

9. A method according to claim 6 or claim 7 leading to the obtention of α-halo α-ethylene aldehyde of the formula 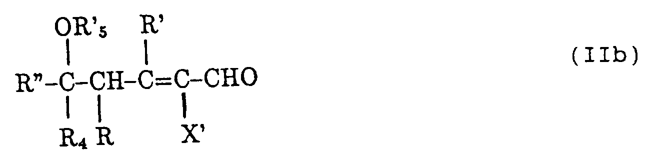 in which R'₅ is a methyl or ethyl radical and the substituents R, R', R'', R₄ and X' satisfy the definitions given in claim 5, characterized by subjecting a dienoxysilane of formula I' to the action of an acetal of the formula 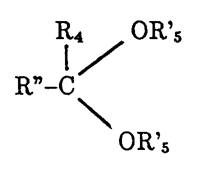 in which R'₅ is a methyl or ethyl radical and the substituents R'' and R₄ satisfy the definitions given in claim 5.

10. A method according to claim 6 or claim 7, leading to the obtention of an α-halo α-ethylene aldehyde of the formula 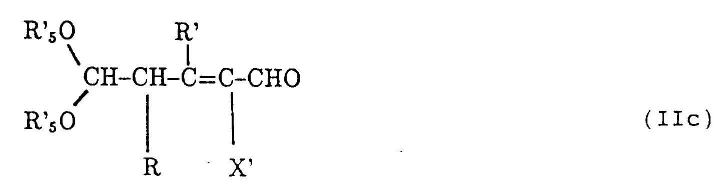 in which R'₅ is a methyl or ethyl radical and the substituents R, R' and X' satisfy the definitions given in claim 5, characterized by subjecting a dienoxysilane of formula I' to the action of an orthoformate of the formula HC(OR'₅)₃ in which R'₅ is a methyl or ethyl radical.

## Patentansprüche

1. Neues Dienoxysilan der allgemeinen Strukturformel 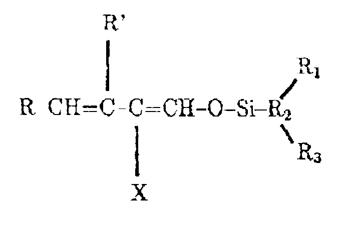 worin R für ein Wasserstoffatom oder ein lineares Alkylradikal mit 1 bis 4 Kohlenstoffatomen steht, R' für ein Wasserstoffatom oder ein Methylradikal steht, R₁, R₂, R₃ jeweils für ein Alkylradikal mit 1 bis 4 Kohlenstoffatomen oder ein Arylradikal stehen und X für ein Halogenatom, ein Dialkylaminradikal, dessen Alkylgruppen jeweils 1 bis 4 Kohlenstoffatome enthalten oder ein Pyrrolidin-, Piperidin- oder Morpholinradikal steht, wobei R' nur dann ein Methylradikal sein kann, wenn R ein Wasserstoffatom ist.

2. Verfahren zur Herstellung eines Dienoxysilans nach Anspruch 1, **dadurch gekennzeichnet**, daß ein α-substituiertes α-Äthylenaldehyd der Strukturformel 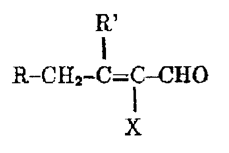 einer konjugierten Reaktion mit Natruiumjodid und einem Chlorsilan der Strukturformel 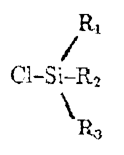 unterworfen wird, wobei die verschiedenen Substituenten dieser beiden Strukturformeln den im Anspruch 1 gegebenen Definitionen entsprechen, die Reaktion bei Raumtemperatur in wasserfreier Umgebung in einer inerten Atmosphäre und unter Bewegung im Beisein einer schwachen Base stattfindet, welche beispielsweise ein tertiäres Amin sein kann, und das Dienoxysilan durch Extraktion mit Hilfe eines geeigneten unpolaren Lösungsmittels aus der Reaktionsumgebung isoliert wird.

3. Verfahren nach Anspruch 2, **dadurch gekennzeichnet**, daß die Reaktion in wasserfreiem Acetonitril und in Gegenwart von Triäthylamin stattfindet.

4. Verfahren nach einem der Ansprüche 2 oder 3, **dadurch gekennzeichnet**, daß das Dienoxysilan aus der Reaktionsmischung durch Extraktion isoliert wird unter Zuhilfenahme eines Lösungsmittels aus der durch Petroläther, Pentan, Hexan und Cyklohexan gebildeten Gruppe.

5. Neues α-halogeniertes α-Äthylenaldehyd, welches ausgehend von einem Dienoxysilan nach Anspruch 1 gewonnen wird, und die allgemeine Strukturformel 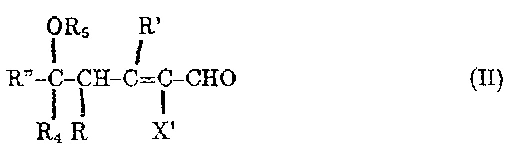 aufweist, worin R für ein Wasserstoffatom oder ein lineares Alkylradikal mit 1 bis 4 Kohlenstoffatomen steht, R' für eine Wasserstoffatom oder ein Methylradikal steht, X' für ein Halogenatom steht, R'' für ein Alkyl-, Aryl-, Heteroaryl-, Alkyläthylen- oder Aryläthylenradikal oder ein Methoxy- oder Ethoxyradikal steht, R₄ für ein Wasserstoffatom oder ein Methylradikal und R₅ für ein Wasserstoffatom oder ein Methyl- oder Ethylradikal steht, wobei R' nur dann ein Methylradikal sein kann, wenn R ein Wasserstoffatom ist.

6. Verfahren zur Herstellung eines α-halogenierten α-Äthylenaldehyds nach Anspruch 5, **dadurch gekennzeichnet**, daß ein Aldehyd, ein Acetal oder ein Orthoformiat mit einem halogenierten Dienoxysilan der Strukturformel 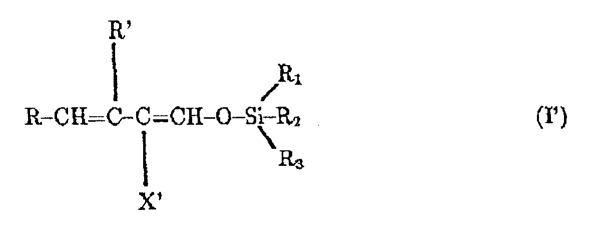 zur Reaktion gebracht wird, wobei X' für ein Halogenatom steht und die Substituenten R, R', R₁, R₂ und R₃ den im Anspruch 1 gegebenen Definitionen entsprechen, die Reaktion bei einer Temperatur von 0°C bis 20°C in einem wasserfreien Lösungsmittel unter inerter Atmosphäre und in Gegenwart eines Lewis-Säurekatalysators stattfindet und das α-halogenierte α-Äthylenaldehyd aus der Reaktionsmischung isoliert wird, indem Wasser zugegeben, die wässrige Phase separiert und die organische Phase auf wasserfreiem Magnesiumsulfat getrocknet wird bevor das Lösungsmittel verdampft wird.

7. Verfahren nach Anspruch 6, **dadurch gekennzeichnet**, daß die Reaktion in wasserfreiem Dichlormethan in Gegenwart eines Katalysators stattfindet, welcher aus der Gruppe Titantetrachlor, Zinkdichlor und Zinkdibrom ausgewählt ist.

8. Verfahren nach einem der Ansprüche 6 oder 7 zur Herstellung eines α-halogenierten α-Äthylenaldehyds der Strukturformel 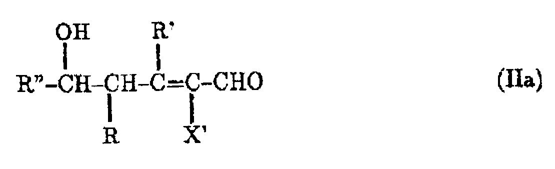 wobei die Substituenten R, R', R'' und X' den in Anspruch 5 genannten Definitionen entsprechen, **dadurch gekennzeichnet**, daß man ein Dienoxysilan nach der Formel (I') mit einem Aldehyd der Formel R''CHO reagieren läßt und das erhaltene Produkt gegebenenfalls einer Hydrolyse unterwirft, um das entsprechende Siloxan in α-halogeniertes α-Äthylenaldehyd umzuwandeln.

9. Verfahren nach einem der Ansprüche 6 oder 7 zur Herstellung eines α-halogenierten α-Äthylenaldehyds der Strukturformel 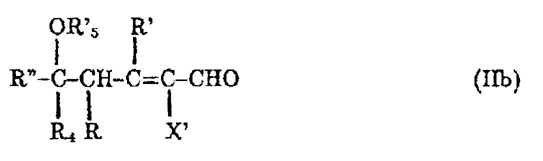 worin R'₅ für ein Methyl- oder Äthylradikal steht und die Substituenten R, R', R'', R₄ und X' den in Anspruch 5 gegebenen Definitionen entsprechen, **dadurch gekennzeichnet**, daß man ein Dienoxysilan gemäß Formel (I') mit einem Acetal der Strukturformel 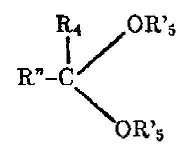 reagieren läßt, wobei R'₅ für ein Methyl- oder Äthylradikal steht und die Substituenten R'' und R₄ den in Anspruch 5 gegebenen Definitionen entsprechen.

10. Verfahren nach einem der Ansprüche 6 oder 7 zur Herstellung eines α-halogenierten α-Äthylenaldehyds der Strukturformel 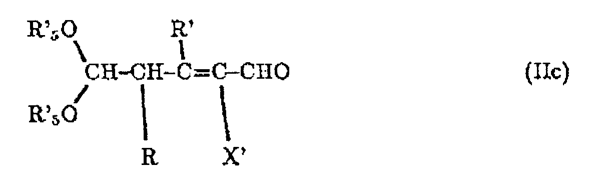 wobei R'₅ für ein Methyl- oder Äthylradikal steht und die Substituenten R, R' und X' den im Anspruch 5 gegebenen Definitionen entsprechen, **dadurch gekennzeichnet**, daß man ein Dienoxysilan gemäß Formel (I') mit einem Orthoformiat der Formel HC(OR'₅)₃ reagieren läßt, wobei R'₅ für ein Methyl- oder Äthylradikal steht.
